# EUROPÄISCHE PATENTSCHRIFT

(11) **EP 0 447 014 B1**
(45) Veröffentlichungstag und Bekanntmachung des Hinweises auf die Patenterteilung: **02.11.1995**
(21) Anmeldenummer: 91250077.4
(22) Anmeldetag: 15.03.1991
(51) Int. Cl.: C07J 41/00, C07J 43/00, C07J 21/00

(54) **Verfahren zur Herstellung von Zwischenprodukten für die Antigestagensynthese (Onapristonsynthese)**
Process for the preparation of intermediates used in the synthesis of progesterone antagonists (synthesis of onapristone)
Procédé pour la préparation des intermédiares utilisés dans la synthèse des composés avec activité anti-progesterone (synthèse d'onapristone)

(30) Priorität: 15.03.1990 DE 4008584
(43) Veröffentlichungstag der Anmeldung: 18.09.1991
(73) Patentinhaber: SCHERING AKTIENGESELLSCHAFT, 13342 Berlin (DE)
(72) Erfinder: Ottow, Eckard, Dr., W-1000 Berlin 45 (DE); Neef, Günter, Dr., W-1000 Berlin 31 (DE); Rohde, Ralph, Dr., W-1000 Berlin 45 (DE)

(56) Entgegenhaltungen:
- EP-A- 0 097 572
- EP-A- 0 110 434
- EP-A- 0 129 499
- EP-A- 0 245 170
- EP-A- 0 305 242
- EP-A- 0 308 345
- WO-A-83/03099

## Beschreibung

Die vorliegende Erfindung betrifft ein neues Verfahren zur Herstellung von Zwischenprodukten der allgemeinen Formel III 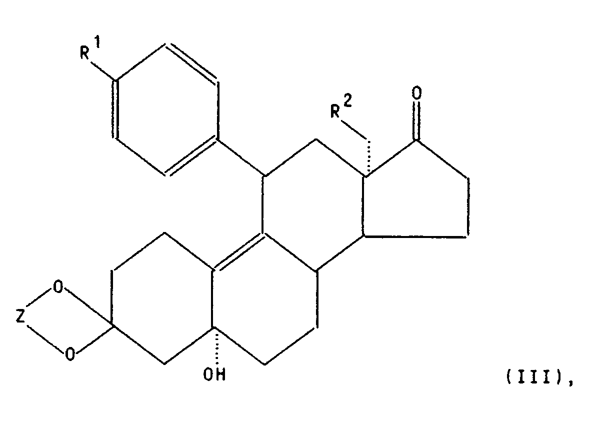 worin
_{R1} 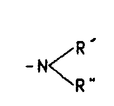 mit R' und R" in der Bedeutung von Wasserstoff oder Alkyl mit 1 bis 4 Kohlenstoffatomen oder R' und R" unter Einschluss von N in der Bedeutung eines gesättigten 5- oder 6-gliedrigen Ringes, wobei im Ring ausser N noch ein weiteres Heteroatom wie O, N, S enthalten sein kann, sowie die entsprechenden tertiären N-Oxide und die Säureadditionssalze, -OR'" mit R"' in der Bedeutung von Methyl, Ethyl, Propyl, Methoxyphenyl, Allyl oder β-Dimethylaminoethyl,
R² ein Wasserstoffatom, eine Methyl- oder Ethylgruppe, und
Z eine Ethylen-oder 2,2-Dimethylpropylengruppe bedeuten.
Die Verbindungen (Zwischenprodukte) der allgemeinen Formel III sind bekannt.
Diese Zwischenprodukte werden benötigt zur Herstellung von Antigestagenen (kompetitiven Progesteronantagonisten) der allgemeinen Formel I 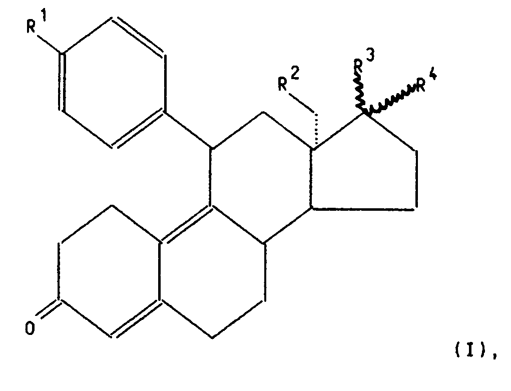 worin
R¹ und R² die in Formel III angegebene Bedeutung haben sowie
^{R3} -(CH₂)ₙ-CH₃ mit n=0-4, -(CH₂)ₙ-CH₂-O(S)R^{IV} mit n = 0-4 und R^{IV} in der Bedeutung von Wasserstoff oder Alkyl mit 1 bis 4 Kohlenstoffatomen, -CH = CH-(CH₂)ₙ-OR^{V} mit n = 1-4 und R^{V} in der Bedeutung von Wasserstoff, Alkyl oder Alkanoyl mit jeweils 1 bis 4 Kohlenstoffatomen, -C=C-X mit X in der Bedeutung von Wasserstoff, Alkyl mit 1 bis 4 Kohlenstoffatomen oder Halogen, -(CH₂)ₙ-CH₂CN mit n = 0-3 oder 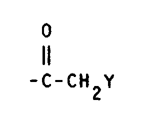 mit Y in der Bedeutung von Wasserstoff oder OR^{V} mit R^{V} in der oben angegebenen Bedeutung,
R⁴ Hydroxy, Alkyloxy oder Alkanoyloxy mit jeweils 1 bis 4 Kohlenstoffatomen oder 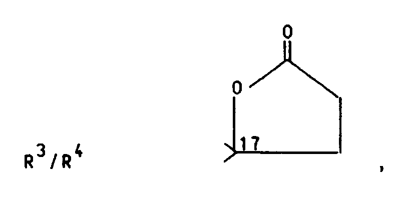 wobei
R³ in a-Stellung und R⁴ in β-Stellung oder R³ in β-Stellung und R⁴ in a-Stellung zum Steroidgerüst stehen, bedeuten.

Diese Antigestagene der Formel I und ein Verfahren zu ihrer Herstellung sind in der Europäischen Patentschrift 0129 499 beschrieben. Das 11 β-(4-Dimethylaminophenyl)-17a-hydroxy-17β-(3-hydroxypropyl)-13a-methyl-4,9-gonadien-3-on (Onapriston) hat sich bisher als besonders vorteilhaft erwiesen (Schneider et al., Eur. J. Cancer Clin. Oncol., 25, S. 691-701 (1989). Als Indikationsgebiete seien insbesondere die Tumortherapie (Mammacarcinom), die Verwendung bei der Geburtseinleitung sowie die Behandlung der Endometriose genannt.

Ein weiteres Verfahren zur Herstellung von Zwischenprodukten der allgemeinen Formel II 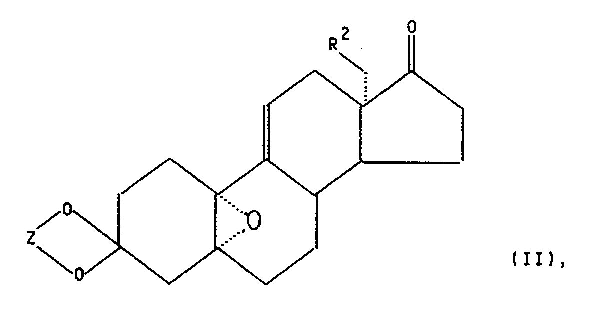 worin
Z und R² die in Formel III angegebene Bedeutung haben,
geht aus der Europäischen Patentanmeldung 0259 248 hervor. Beide bekannten Verfahren gehen aus von der Verbindung 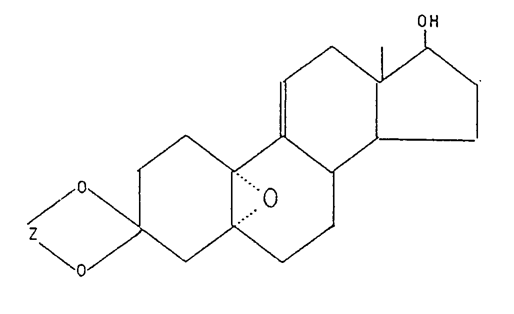 worin Z die in Formel III angegebene Bedeutung hat.
Die Verbindung der Formel III kann beispielsweise nach dem in der EP-A 0298020 beschriebenen Verfahren hergestellt werden.

In der Abbildung 1 sind die beiden bekannten Verfahren zur Herstellung von Antigestagenen der Formel I am Beispiel der Synthese von Onapriston einander gegenübergestellt; THP steht dabei für den 2-Tetrahydropyranyl-Rest.

Die Reaktionsfolge 5 → 6 → 7 → 8 → 9 ist in der Europäischen Patentschrift 0 129 499, die Sequenz 5 → 13 → 14 → 15 → @ der EP-A 0 259 248 beschrieben.

Beim erfindungsgemäßen Verfahren zur Herstellung der Zwischenprodukte der allgemeinen Formel III wird nunmehr eine Verbindung der allgemeinen Formel II mit einer Verbindung der Formel 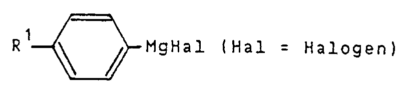 unter Zusatz eines Cu(I)-Salzes, wie z.B. CuCI, Cul oder CuCN, umgesetzt.

Die Einführung eines substituierten Arylrestes in 3,3-Diacetal-5a,10a-epoxy-estr-9(11)-en-dione in der 11β-Position - also im Falle eines β-ständigen 13-Allylsubstituenten - durch Addition eines substituierten Arylmagnesiumhalogenids oder einer Aryllithiumverbindung unter Kupfer-(I)-katalyse geht bereits aus den EP-AS 0 245 170, 0 305 242, 0 697 572, 0 308 345 und 0 110 434 sowie der WO-A 83/03099 hervor. 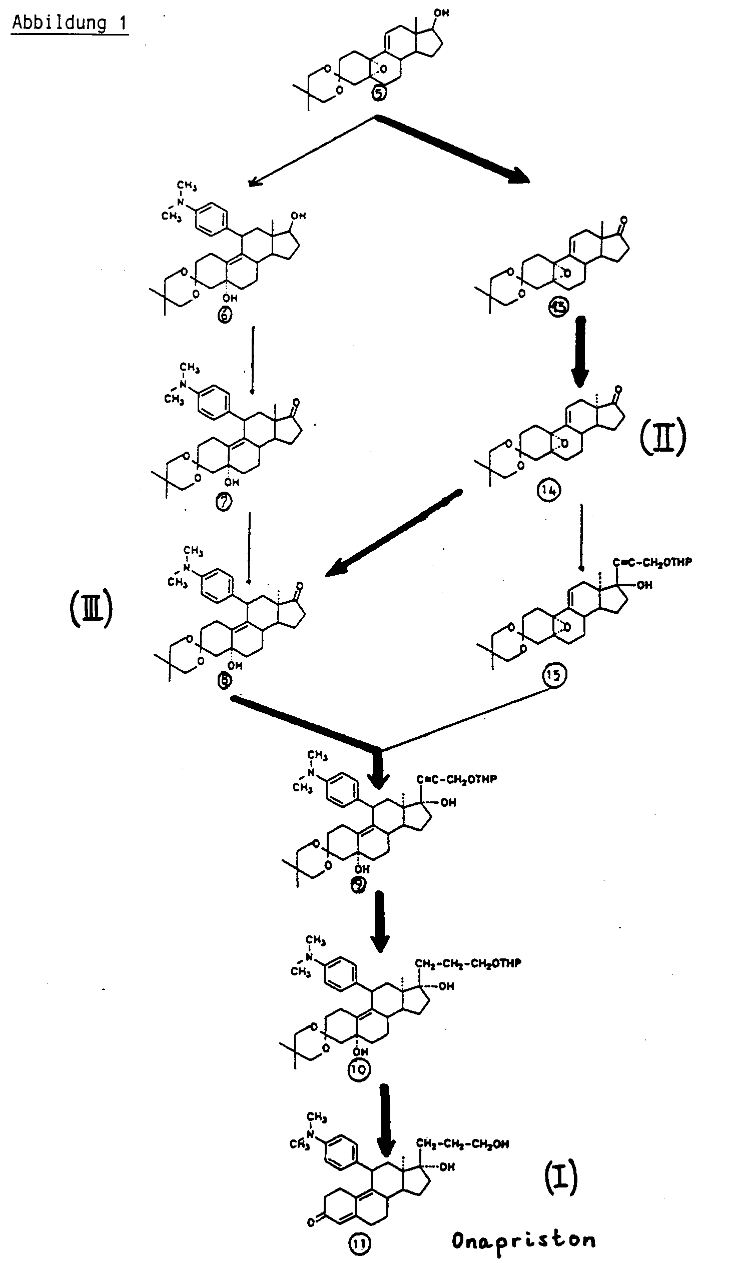 Die so erhaltene Verbindung der allgemeinen Formel III wird dann nach der in der EP 0129 499 angegebenen Art und Weise zu einem Endprodukt der allgemeinen Formel I weiterverarbeitet. (in Abb. 1 8 → 9 → 10 → 11).

Der erfolgreiche Verlauf der Addition des nucleophilen Reagenzes ausschließlich an die Epoxyfunktion der Verbindung der allgemeinen Formel II ist überraschend. Eigentlich wäre auch der nucleophile Angriff an der 17-Ketofunktion zu erwarten gewesen.

Gegenüber den bekannten Verfahren bietet das erfindungsgemäße Verfahren entscheidende Vorteile. Zwar läßt sich bei der nucleophilen Epoxidöffnung 5 → @ gemäß EP-A 0259 248 eine Ausbeute von 84,3 % erreichen; es hat sich aber herausgestellt, daß @(sowie dessen Hydrierungsprodukt) in beträchtlichem Maße zur Zersetzung neigt und deshalb nicht lagerstabil ist. Dies fällt insbesondere bei der Durchführung technischer Ansätze negativ ins Gewicht, wenn nämlich eine Zwischenlagerung größerer Mengen @ erforderlich ist.

Die Gesamtausbeute kann erhöht werden, wenn unumgesetztes Material der Formel 13 nach der Bestrahlung zur Isomerisierung der 13ß-Methyl- oder Ethylgruppe zurückgewonnen und zur erneuten Bestrahlung wiederverwendet wird. Die Rückgewinnung ist im Beispiel "Herstellung des Ausgangsproduktes" beschrieben.

Bei der Herstellung von 8 über die in der EP-A 0129 499 beschriebene Reaktionssequenz kann die Oxidation der 17ß-Hydroxy- zur 17-Ketogruppe (6 → 7) nur unter den Bedingungen einer Oppenauer-Oxidation durchgeführt werden, damit eine Mitoxidation der Dimethylaminofunktion verhindert wird. Da es sich bei der Oppenauer-Oxidation um eine typische Gleichgewichtsreaktion handelt, wird die in Steroids 44 (1989). S. 368 angegebene Ausbeute von 80,5% nur durch Zusatz einer Ketokomponente (Cyclohexanon in siedendem Toluol) im Überschuß erzielt; die Ketokomponente bewirkt eine Verschiebung des Gleichgewichts auf die Seite des gewünschten 17-Keto-Oxidationsproduktes. Diese drastischen, basischen Reaktionsbedingungen führen zu Kondensationsreaktionen der zugesetzten Ketokomponente (Aldoladdition, Knoevenagel-Reaktion). Die dadurch in beträchtlicher Menge entstandenen Nebenprodukte müssen anschließend erst durch Auswaschen, Filtrieren und Chromatographieren wieder abgetrennt werden. Wollte man eine annähernd quantitative Ausbeute erreichen, wäre dies, wenn überhaupt, nur unter Inkaufnahme noch größerer Mengen an schwierig abzutrennenden Nebenprodukten möglich. Zusätzliche Schwierigkeiten ergeben sich wegen der leichten Hydrolisierbarkeit des zu verwendenden Oxidationsmittels Aluminiumtriisopropylat.

Es wurde gefunden, daß die Oxidation in vorteilhafter Weise mit Schwefeltrioxid/Pyridin - wie in Vorschrift I) angegeben - durchgeführt werden kann. Zwar wird dann ebenfalls "nur" eine Ausbeute von ca. 85% erreicht, aber man erhält reines 17-Keton @ ohne - im technischen Maßstab sehr aufwendiges - Chromatographieren.

Das neue Verfahren führt also in vorteilhafter Weise unter Umgehung der die Ausbeute limitierenden und aufwendigen Oppenauer-Oxidation zur Verbindung 8 ( → 9) bzw. unter Umgehung der zersetzlichen Verbindung @ über 8 zu @.

Die nachfolgenden Beispiele dienen der näheren Erläuterung der Erfindung.

### Herstellung des Ausgangsproduktes

### I) 5a,10a-Epoxy-3,3-(2,2-dimethyltrimethylendioxy)-9(11)-estren-17-on

Oxidation von 5 mit Schwefeltrioxid/Pyridin

Eine Suspension von 32,6 g (0,20 Mol) Schwefeltrioxid-Pyridin-Komplex in 25 ml wasserfreiem Dimethylsulfoxid wird auf 10°C gekühlt und unter Schutzgas innerhalb von fünf Minuten mit 30,2 g (0,83 Mol) N-Ethyl-diisopropylamin versetzt. Dabei wird eine schwache Exothermie von 3-5 °C beobachtet. Anschließend wird eine Lösung von 24,8 g (66,2 mMol) 3,3-(2,2-Dimethyltrimethylendioxy)-5a,10a-epoxy-9(10)-estren-17ß-ol in 50 ml wasserfreiem Dimethylsulfoxid innerhalb von 10 Minuten in der Weise zugetropft, daß die Temperatur des Reaktionsgemisches 25 ° C nicht übersteigt. Danach wird eine Stunde bei Raumtemperatur gerührt. Nach Dünnschichtchromatographie-Kontrolle auf vollständige Umsetzung (Kieselgelso-Fertigplatten/Merck; n-Hexan/Ethylacetat = 1/1) werden 100 ml Ethylacetat zugegeben. Danach wird das Reaktionsgemisch auf 10_{°}C gekühlt und innerhalb von 15 Minuten vorsichtig mit 100 ml eiskaltem Wasser versetzt, so, daß die Temperatur nicht über 20 ° C ansteigt. Nach 15minütigem Nachrühren werden die Phasen getrennt. Die Wasserphase wird mit 100 ml Ethylacetat nachextrahiert. Die vereinigten Ethylacetatphasen werden zweimal mit je 50 ml Wasser gewaschen, über 25 g Natriumsulfat getrocknet und filtriert. Die filtrierte Ethylacetatlösung wird unter sukzessiver Zugabe von n-Hexan kodestilliert, bis ein Siedepunkt von 68 ° C (reines n-Hexan) erreicht ist. Anschließend wird auf 0 ° C gekühlt und 15 Minuten nachgerührt. Der gebildete farblose Niederschlag wird abgesaugt und bis zur Gewichtskonstanz getrocknet. Das so erhaltene Rohprodukt (27,6 g; Ausbeute > Theorie) wird durch Erhitzen mit 55 ml n-Hexan aufgereinigt. Man erhält 20,98 g (85,1%) der Titelverbindung mit einem Schmelzpunkt von 125-127 ° C.

### 11) 5a,10a-Epoxy-3,3-(2,2-dimethyltrimethylendioxy)-13a-methyl-9(11)-gonen-17-on

25 g 5a,10a-Epoxy-3,3-(2,2-dimethyltrimethylendioxy)-9(11)-estren-17-on werden unter Schutzgas gelöst in 6 I Hexan 3,5 Stunden bei Raumtemperatur mit einem Hg-Hochdruckstrahler (Heraeus, TQ 150, 150 W) in einer Quarzglas-Tauchapparatur bestrahlt. Nach Abzug des Solvens am Vakuum wird der Rückstand an Aluminiumoxid (neutral, Stufe 111) mit einem Gemisch aus Ethylacetat und Hexan chromatographiert. Es werden 10,75 g 5a,10a-Epoxy-3,3-(2,2-dimethyltrimethylendioxy)-13a-methyl-9(11)-gonen-17-on und 8 g Ausgangsmaterial als weiße Schäume isoliert.
10,75 g = 43% (63% bezogen auf umgesetztes Startmaterial).

### Beispiel 1

### 11β-(4-Dimethylaminophenyl)-3,3-(2,2-dimethyltrimethylendioxy)-5α-hydroxy -13A-methyl-9-gonen-17-on

2,63 g Magnesiumspäne werden bei Raumtemperatur unter Schutzgas in 20 ml absolutem Tetrahydrofuran vorgelegt und zunächst mit 0,3 ml 1,2-Dibromethan versetzt. Nach erfolgter Aktivierung des Magnesiums werden 21,5 g 4-Brom-N,N-dimethylanilin gelöst in 200 ml absolutem Tetrahydrofuran so zugetropft daß die Innentemperatur im Reaktionsgefäß nicht über 40 °C steigt. steigt. Nach erfolgter Bildung der Arylmagnesiumverbindung wird das Reaktionsgemisch auf -10 ° C abgekühlt, mit 270 mg Kupfer-(I)-chlorid versetzt und eine Lösung von 5 g 5a,10a-Epoxy-3,3-(2,2-dimethyltrimethylendioxy)13a-methyl-9(11)-gonen-17-on in 70 ml absolutem Tetrahydrofuran langsam zugetropft. Nach viereinhalbstündigem Nachrühren bei -10 bis 0 ° C wird das Reaktionsgemisch auf gesättigte Ammoniumchloridlösung gegossen und die wässrige Phase mit Ethylacetat mehrmals extrahiert. Die vereinigten organischen Phasen werden mit gesättigter Natriumchloridlösung neutralgewaschen, über Natriumsulfat getrocknet und am Vakuum eingeengt. Der Rückstand wird an Kieselgel mit einem Gemisch aus Ethylacetat/Hexan chromatographiert. Es werden 6,42g 11β-(4-Dimethylaminophenyl)-3,3-(2,2-dimethyltrimethylendioxy)-5a-hydroxy-13a-methyl-9-gonen-17- on als weißer Schaum erhalten. 6,42 g = 96,9%.

## Patentansprüche

1. Verfahren zur Herstellung von Zwischenprodukten der allgemeinen Formel III 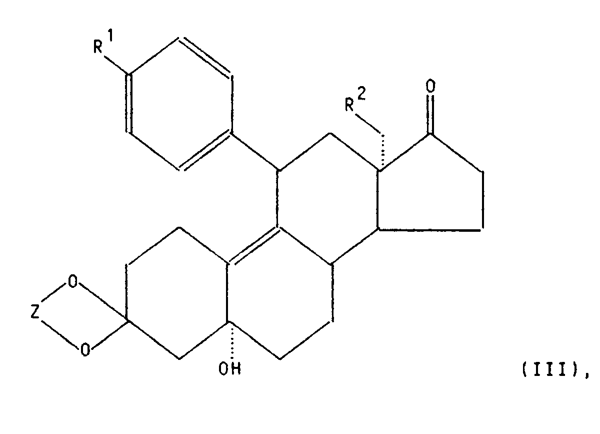 worin
_{R1} 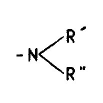 mit R' und R" in der Bedeutung von Wasserstoff oder Alkyl mit 1 bis 4 Kohlenstoffatomen oder R' und R" unter Einschluss von N in der Bedeutung eines gesättigten 5- oder 6- gliedrigen Ringes, wobei im Ring ausser N noch ein weiteres Heteroatom wie O, N, S enthalten sein kann, sowie die entsprechenden tertiären N-Oxide und die Säureadditionssalze, -OR'" mit R"' in der Bedeutung von Methyl, Ethyl, Propyl, Methoxyphenyl, Allyl oder β-Dimethylaminoethyl,
R² ein Wasserstoffatom, eine Methyl- oder Ethylgruppe,
Z eine Ethylen-oder 2,2-Dimethylpropylengruppe bedeuten, dadurch gekennzeichnet, daß eine Verbindung der allgemeinen Formel II 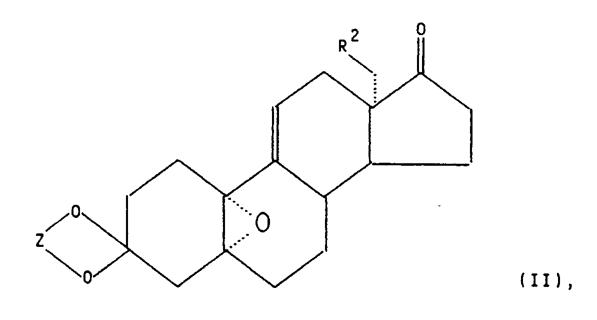 worin
R² und Z die in Formel III angegebene Bedeutung haben, mit einer Verbindung der Formel 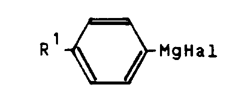 (Hal = Halogen) unter Zusatz eines Cu(I)-Salzes umgesetzt wird.

## Claims

1. Process for the preparation of intermediates of the general formula III 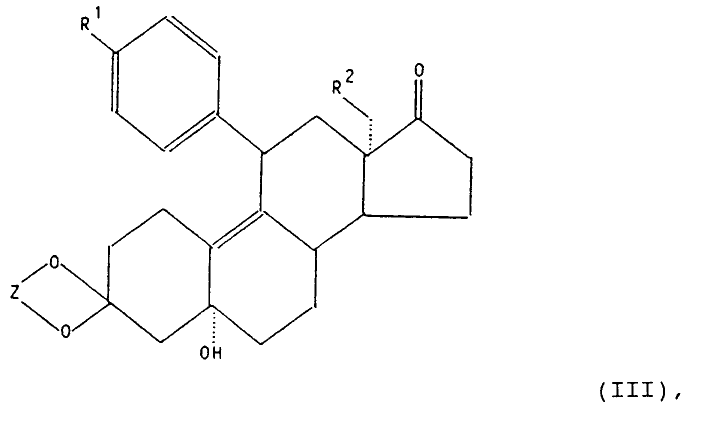 wherein
R¹ represents 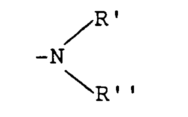 wherein R' and R" are hydrogen or alkyl having from 1 to 4 carbon atoms, or R' and R" together with N form a saturated 5- or 6-membered ring which in addition to N may contain a further hetero atom, such as O, N or S, and also the corresponding tertiary N-oxides and the acid addition salts, or -OR'" wherein R"' is methyl, ethyl, propyl, methoxyphenyl, allyl or β-dimethylaminoethyl,
R² represents a hydrogen atom or a methyl or ethyl group, and
Z represents an ethylene or 2,2-dimethylpropylene group, characterised in that a compound of the general formula II 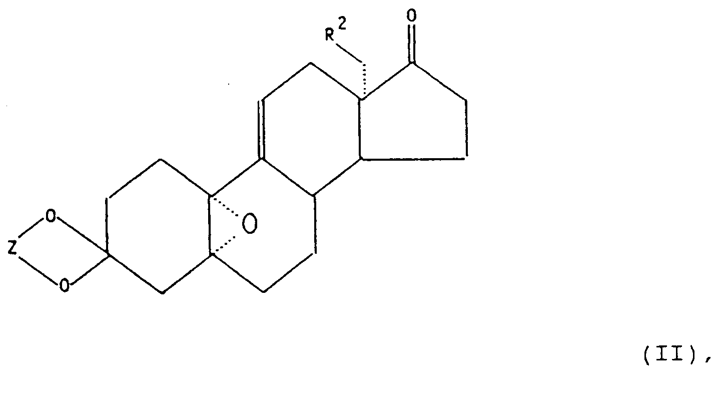 wherein
R² and Z have the meanings given in formula III,
is reacted with a compound of the formula 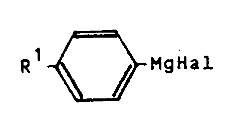 (Hal = Halogen)
with the addition of a Cu(I) salt.

## Revendications

1. Procédé de préparation des produits intermédiaires de formule générale III 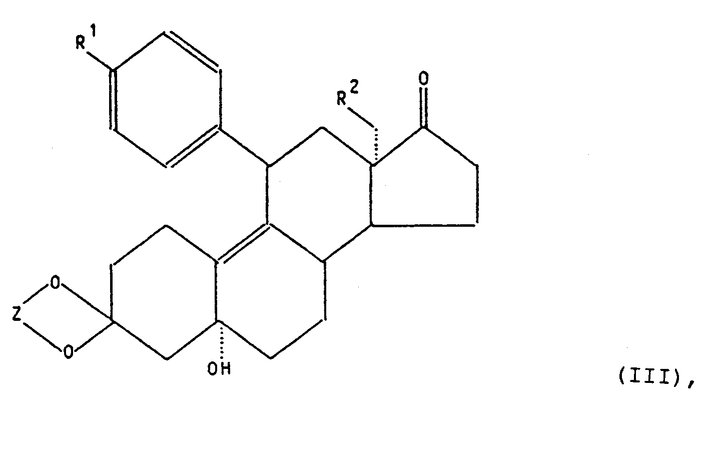 dans laquelle
R¹ représente un groupe 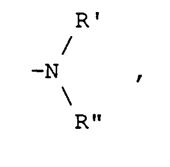 R' et R" étant chacun l'hydrogène ou un alkyle avec de 1 à 4 atomes de carbone ou bien formant en incluant l'atome d'azote un cycle saturé à 5 ou 6 chaînons, pouvant encore comprendre en plus de l'azote un autre hétéroatome tel que O, N ou S, ainsi que les N-oxydes tertiaires et sels d'addition d'acides correspondants,
un groupe OR'" avec R"' désignant un groupe méthyle, éthyle, propyle, méthoxyphényle, allyle ou β-diméthylaminoéthyle,
R² représente un atome d'hydrogène, un groupe méthyle ou éthyle
Z représente un groupe éthylène ou 2,2-diméthylpropylène,
procédé caractérisé en ce que l'on fait réagir un composé de formule générale II 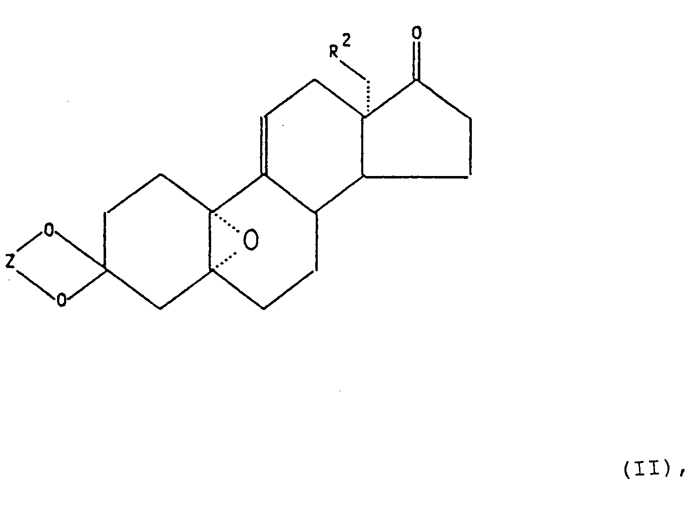 dans laquelle
R^{z} et Z ont la signification donnée dans la formule III avec un composé de formule 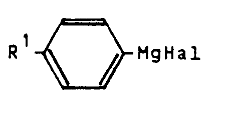 (Hal désignant un halogène) en présence
d'un sel de Cu(I).
